# EUROPEAN PATENT APPLICATION

(11) **EP 3 739 064 A1**
(43) Date of publication of application: **18.11.2020**
(21) Application number: 19174784.9
(22) Date of filing: 15.05.2019
(51) Int. Cl.: C12Q 1/6827, C12Q 1/6851

(54) **COMPARATIVE ANALYSIS OF MICROSATELLITES BY CAPILLARY ELECTROPHORESIS (CE) DNA PROFILES**

(71) Applicant: Biotype GmbH, 01109 Dresden (DE)
(72) Inventor: Tabiti, Karim, 01109 Dresden (DE)
(74) Representative: Heide, Anna Katharina

(57) **Abstract**

The present invention is directed to a method for determining of at least one microsatellite instability (MSI) based on a shift in a capillary electrophoresis (CE) profile (CE profile shift), the CE profile shift being determined by a comparison between the capillary electrophoresis (CE) profile of a target sequence of at least one microsatellite (MSI target profile) and the capillary electrophoresis (CE) profile of its specific wild type sequence (MS wild type profile). Further, the invention encompass suitable primer for use in said method, a kit comprising all essential components for performing said method successfully, a complete closed device as a system, namely "MSI Modaplex Analysis System" and a method for diagnosis of MSI phenotypes associated with an inflammation, cancer, inflammation associated cancer and/or auto immune disease, wherein the diagnosis comprises the method for determining of at least one CE profile shift as mentioned above. Finally, the present invention is directed to the use of an improved MSI panel for the determination and preferably diagnosis of MSI tumors, said panel consist of the STR biomarker NR-21, NR-24, Mono27, D2S123, D5S346, D17S250, Bat-25 and Bat-26.

## Description

The present invention is directed to a method for determining of at least one microsatellite instability (MSI) based on a shift in a capillary electrophoresis (CE) profile (CE profile shift), the CE profile shift being determined by a comparison between the capillary electrophoresis (CE) profile of a target sequence of at least one microsatellite (MSI target profile) and the capillary electrophoresis (CE) profile of its specific wild type sequence (MS wild type profile). Further, the invention encompass suitable primer for use in said method, a kit comprising all essential components for performing said method successfully, a complete closed device as a system, namely "MSI Modaplex Analysis System" and a method for diagnosis of MSI phenotypes associated with an inflammation, cancer, inflammation associated cancer and/or auto immune disease, wherein the diagnosis comprises the method for determining of at least one CE profile shift as mentioned above. Finally, the present invention is directed to the use of an improved MSI panel for the determination and preferably diagnosis of MSI tumors, said panel consist of the STR biomarker NR-21, NR-24, Mono27, D2S123, D5S346, D17S250, Bat-25 and Bat-26.

In the prior art various methods are disclosed for identification of microsatellite instabilities (MSI). One method for evaluating microsatellite instabilities which are associated with a tumor, is disclosed in EP1478781, wherein the microsatellite loci in a biological sample comprising genomic DNA from a tumor are amplified, the sizes of the DNA amplification products are determined and then the size of the tumoral amplification product and the size of the normal amplification product are compared. Where for a tumoral DNA amplification product a size is confirmed which is at least 3 bp or 2 bp shorter than the respective normal amplification product an MSI is evaluated. Said method is disclosed for the biomarker Bat-26, Bat-25, NR-21, NR-22, NR-24 and NR-27. The disadvantage of said method is the precondition of the aforementioned resolution for the evaluation of an MSI. WO02086448A2 discloses a method for detection of MSI and its uses in diagnosis of tumors with different biomarker. The disadvantage of said method is that the user or physician has to deal with stutter artefacts making the diagnosis of a MSI comparatively difficult.

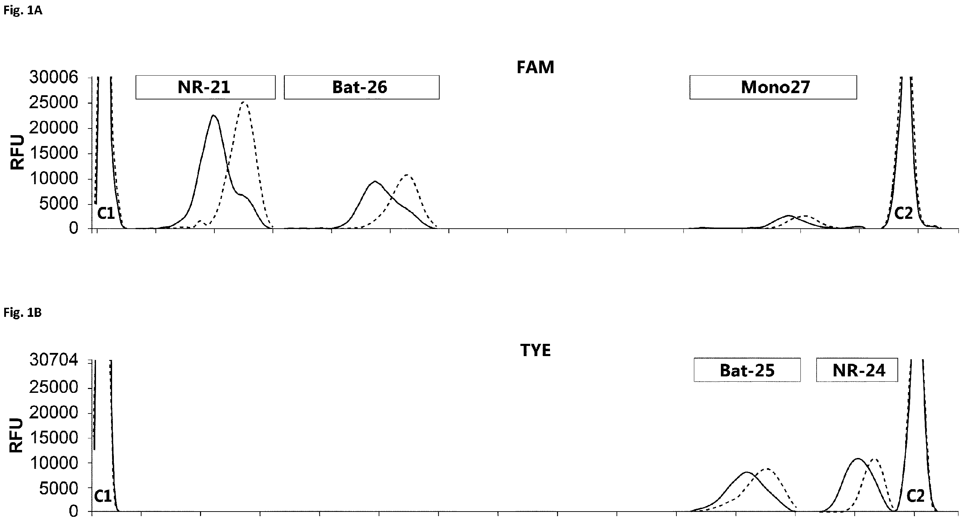

Therefore the applicant developed based on the Modaplex technology, which in turn is a method based on a real-time PCR in combination with capillary gel electrophoresis, a novel method overcoming the disadvantages and combining more biomarker for a valid determination of a MSI and potentially for a valid diagnosis. Additionally, a fully closed device is provided. The distinguishing feature compared to the prior art is that determination of an MSI and identification of said MSI is performed based of the migration profile of amplicons achieved during said real time PCR and which are not resolved to single molecule peaks within the CE electropherogram. According to the invention stutter artefacts are not distinguished. Thus, the evidence for the presence of an MSI is not the identified sequence and not its size [bp] but the behavior of the amplicons of the potential MSI target sequence and the behavior of the amplicons of the respective wildtype sequence while real time PCR and CE. Said behavior is demonstrated by the migration trough the capillary under certain conditions and said migration is reflected by the profile of the respective amplicons as shown for example in Fig.1A and B. The following comparison of both profiles can be an evidence for an MSI in case a shift between the profiles is identified.

Thus, the present invention combines the advantages of a real time PCR and the capillary electrophoresis (synonym capillary gel electrophoresis) but without resolution and sequencing of the achieved amplicons to the single base.

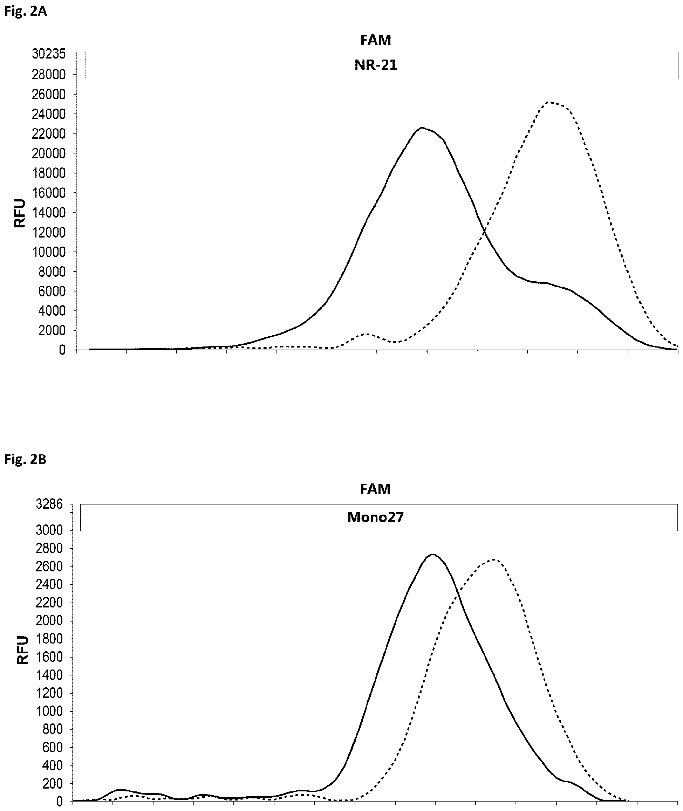

Another distinguishing feature of the present invention compared to the prior art is that in case of STRs, the inventive DNA profiles comprise the sum of all data-pairs (RFUs over electrophoretic analyte retention times) of true STR alleles (up to 2 for heterozygous wildtype DNA) and their corresponding stutter artefacts. The calculated DNA profiles of altered STRs - target profile or target modaplex profile - can be only interpreted and quantified in comparison to a wildtype profile or wildtype modaplex profile as defined herein (e. g. wildtype nucleic acid in case of mutations). Further, the stutter artefacts as known from prior art are not resolved in the inventive profile as shown for example in Fig. 2A, making determination and diagnosis of a MSI much easier for a user

A further advantage of the present invention is that it provides a complete closed system. The PCR is performed in the defined Modaplex device so that no transfer of any samples is necessary. Therefore, contamination due to sample transfer can be avoided.

Because, sequencing is not necessary the inventive method is faster and provides an earlier results for which only one device, namely the Modaplex device, is necessary. The inventive method avoids the expenses and the time to be invested to purchase a DNA Sequencing device or for the external sequencing of the samples.

Therefore, the object of the present invention is to provide
- a fast method for the determination of a potential mutation in a MSI avoiding sequencing,
- a closed and automatically operating system avoiding contamination and avoiding various single (hands-on) steps and hands-on time,
- all components for performing the above defined method which are also suitable for use in such a device, therefore it is an object to provide a complete independent system.
- An another object is to provide a method, system and device for a multiplex analysis of different MSI, in particular for research, pre-clinical studies and diagnosis,
- A further object is to provide a method, primer, kit and device for the determination of MSI according to the Bethesda panel and revised Bethesda panel without sequencing,
- to provide an improved biomarker panel for a valid MSI determination and preferably valid diagnosis of MSI tumors and
- a system for the determination of novel STR biomarker associated with an inflammation, cancer, inflammation associated cancer and/or auto immune disease, in particular in research and diagnosis, and
- an easy handle system comprising a method, the relevant components as primers and controls, the device and a software to support the use for a significant analysis and preferably diagnosis of MSI.

Therefore, in order to solve the above objects the present invention provides the subject matters as defined within the claims and detailed explained in the following. The feasibility is shown by the selected examples which are not to be interpreted limiting the scope of protection.

Therefore a first aspect of the of the solution according to the resent invention is a method for determining of at least one microsatellite instability (MSI) based on a shift in a capillary electrophoresis (CE) profile (CE profile shift = profile shift), the CE profile shift being determined by a comparison between the capillary electrophoresis (CE) profile of a target sequence of at least one microsatellite (MSI target CE profile = target profile) and the capillary electrophoresis (CE) profile of its specific wild type sequence (MS wild type CE profile = wild type pofile), the method comprises the steps of
- amplification by means of a real time PCR simultaneously of at least one target sequence and of at least one specific wild type sequence, in particular representing at least one STR Biomarker, and labeling nucleic acids thereof during amplification, in particular achieving amplicons with a **label** or **detectable label,**
- separating the labeled nucleic acids, in particular of the obtained amplicons, by capillary electrophoresis (CE), in particular comprising electrokinetically injection, and detecting at least one, fluorophore labeled amplicon of at least one target sequence and of at least one wild type sequence, respectively, in real time while amplification,
- computer program based calculation of the at least one target profile and of the at least one corresponding wild type profile,
- comparing the at least one target profile and the corresponding wild type profile, in particular for direct comparison an overlay of the achieved target profile and wild type profile is performed manually or preferably automatically by a suitable software (computer program) and
- determining a CE profile shift between the target profile and the corresponding wild type profile, in particular performed visually by a person who apply said method or by a computer program being written to identify a significant CE profile shift. Preferably, said CE profile shift indicates at least one MSI of interest.

Preferably the at least one microsatellite instability (MSI) is selected from mononucleotide (Bat-25, Bat-26, NR-21, NR-24, NR-22), quasimonomorphic mononucleotide and/or dinucleotide (D5S346, D2S123 and D17S250) repeats. The CE profile shift comprise insertion and deletion.

Detecting primer extension products (amplicons) comprising distinguishably detectably labeled oligonucleotide primer corresponding to each sample-specific sequence tag, wherein said separating nucleic acid molecules and said detecting incorporation is performed in real time during amplification and wherein said detection permits the monitoring of said amplification in real time in CE.

In a preferred embodiment the inventive method comprises or analyzes simultaneously at least three reaction setups for a) at least one MSI target sequence, for b) at least one MS wild type sequence and for c) a calibration sequence (synonym **Modaplex calibrator).** At least one calibration sequence is amplified simultaneously in order to define the range of amplicon detection.

More preferably a forth reaction setup is applied/used in the inventive method simultaneously which is a contamination control (synonym or **patient traceability control**) as defined below. The inventive contamination control, as defined herein is relevant for and known from human DNA profiling in forensics. Within the meaning of the present invention the contamination control fulfilling the function of tractability of a biological samples and the respective target sequence of the selected STR biomarker.

**Target sequences** (synonym for target nucleic acid sequence) are defined by this invention as specific DNA analytes to be identified and/or quantified by the claimed technology or method. For example, the target sequence may lie between the hybridization regions of a pair of oligonucleotide primers which are used to amplify it (defined below). Target sequences comprise human, animal and plant DNA. In particular, the target sequence comprises a sequence which is specific for a biomarker or gene for the identification of a genetically classified or characterized medical indication (disease). A target sequence is a nucleic acid sequence which is specific for a biomarker serving for e. g. specific identification of an individual (forensics, genealogy), of a species (e. g. pathogen, genetically modified organisms), of a disease (e. g. cancer) or of another biological characteristic within the frame of an analytics or diagnostics. Said target sequence is comprised in or obtained from a biological sample (synonymous = specimen, = biological material which is isolated from its natural environment and contains a polynucleotide).

As used herein, **wild type sequence** (synonymous = **wild type biomaterial**) is the non-affected nucleic acid sequence of a biomarker of interested. The wild type sequence can comprise in or be obtained from a biological sample or from a standardized biobank (in context with digital wild type sequences). The wild type sequences are used herein to achieve a wild type profile of said wild type sequence (Biomarker for MSI) in a CE according to the inventive method (synonym "Modaplex- wild type -Profile" vs. "Modaplex- wild type -Profile") as a basis for a comparison of both the "Modaplex- wild type -CE Profile" vs. "Modaplex-Target-CE Profile" and at last for the determination of a shift between said profiles (CE profile shift). In case of an identified shift in the target sequence (analyte or biomarker) of this invention an MSI can be identified and potentially qualified (e. g. MSS = microsatellite stable, MSI-L = microsatellite instability (MSI) MSI low, MSI-H = microsatellite instability MSI high). In a special embodiment the wild type CE profile for at least one biomarker of interest is saved as a digital wild type CE profile within a wild type CE profile data bank which is saved on a storage medium (microchip/memory device etc).

A **biological sample** is the source for the target sequence and can be the source for the wild type sequence. Preferably both - target and wild type sequences - are obtained from the same biological donor (human, animal, plant). A biological sample in turn is or can be obtained from a solid or liquid biopsy and include specimen which predominantly or exclusively contain nucleic acids of benign, otherwise non-affected or in general biological background material which is distinguished from or interferes with the biomarker of interest. Where the biological sample is of human or animal origin it can be obtained by microdissection from microscopically (pathologically) pre-characterized FFPE tissues or otherwise unaffected solid or liquid biopsies (e. g. blood cells versus solid tumor). The biological sample also comprise plants and corresponding target sequences of plant DNA.

The sample comprises any conceivable source materials having biological amount. Preferably the sample according to this invention is a human sample which can be subdivided in solid or liquid biopsies. The most preferential solid biopsies in the diagnosis of human mutations are today formalin-fixed and paraffin-embedded (FFPE) tissues used in pathology. However, this invention is not restricted to this solid sample type. In case of malignant diseases (e. g. cancer) FFPE tissues contain disease affected as well as benign cells in different ratios. Both cell types can be separated by microdissection and the latter cell type can be used as reference biomaterial. Human extracellular circulatory liquids are in particular blood, plasma, serum and/or lymph, digestive juices, in particular saliva, gastric juice, juice of the pancreas and/or gall, secretions and excretions, in particular sweat, urine, feces, ejaculate, vaginal secretions, tear fluids, nasal secretion and/or mother's milk and/or further liquids or secretions, in particular amniotic fluid, cerumen, cerebral fluid. Liquid biopsies contain also in different degrees a mixture of nucleic acids from background and affected cells. The latter can be sometimes enriched as described in the state of the art (e. g. immunoseparation with cell surface specific monoclonal antibodies).

**Biomarkers** (synonym STR biomarker) are defined as nucleic acid analytes (target sequences) which correlate with an organism, virus or biological function. Examples for biomarkers are as such biomarker to differentiate species (e. g. pathogens from hosts organisms) or individuals, strains or traits of a species. Furthermore, biomarkers may correlate with a disease (medical indication) and/or physiological status of a microorganism, human, animal or plant. In case of human beings the latter correlations are also referred to as scientific and/or clinical evidence of an *in vitro* diagnostic device. Microsatellites are useful biomarker for various medical indications - inflammation, cancer, inflammation associated cancer and/or auto immune diseases - or genetic alterations.

Inflammation comprises microorganism associated inflammation caused by virus, bacteria or both. Inflammation also comprise chronic inflammation, e.g. of the gastro intestinal tract, of the upper and lower respiratory tract, asthma, sarcoidosis, of the skin, psoriasis, rheumatic diseases. Preferably, mononucleotide, quasimonomorphic mononucleotide and/or dinucleotide repeats associated with the medical indications described herein are used as biomarker within the inventive methods.

**Microsatellites (synonym STR biomarker),** although referred to as short sequence repeats (SSRs) or short tandem repeats (STRs), are genomic regions of DNA with a repetitive sequence of 1 - 10 bp. Often, microsatellite stretches may be disrupted by base substitutions (imperfect microsatellites) or insertions (interrupted microsatellite). Sometimes, microsatellites may also consist of more than a single repeat type (compound microsatellite) (Schloetterer 2000, Vieira *et* al. 2016). They are frequently found in non-coding DNA regions for proteins or essential RNA (e. g. introns or other "silent" genomic regions) of all organisms and even virus, and are highly polymorphic among individuals of higher organisms or microbial strains (>10 - 20 alleles). However, some of which, especially affecting STRs with repeating units of one or two nucleotides, are described as quasi-monomorphic giving rise to one or only a few alleles within a defined population. Polymorphic STRs have been popularly used as genetic biomarkers for artificial trait selection (e. g. plants or animals breeding; Miah *et al.* 2013, Merritt *et al.* 2015, Zhao *et al.* 2018), epidemiological studies (Klaassen 2009, Chen *et al.* 2011), forensic applications (e. g. genetic fingerprint, paternity test; Becker *et al.* 2007a, Becker *et al.* 2007b, Romos and Vallone 2015) and chimerism analysis after blood stem cell transplantation (Thiede *et al.* 2004). In addition, numerous STRs have been considered as biomarkers for their association with genetic diseases and gene regulatory functions. Quasi-monomorphic STR have been shown to be very informative in case of microsatellite instability (MSI) analysis (Umar *et al.* 2004). Each target sequence is specific for one defined and selected microsatellite which in turn can be used as a biomarker.

**Microsatellite Instability (MSI)** has become a popular research and diagnostic issue for detecting its association with somatic mutations (loss or gain of STR repeat units). STRs with mono- and dinucleotide repeats have proven to be most sensitive due to their quasi-monomorphic occurrence in nature. Several cancers such as colorectal, prostate, gastric, endometrial, ovarian and breast cancers were reported for their association with MSI. It is a surrogate marker caused by deficiency of the DNA mismatch repair (MMR) during DNA replication (also referred to as replication error, RER or mismatch repair deficiency, dMMR), and shows positive predictive value for the efficacy of immune checkpoint inhibitors (ICI) therapy in metastatic tumors (Boland *et al.* 1998, Boland and Goel 2010, Colle *et al.* 2017, Svrcek *et al.* 2019). In addition, some infectious diseases (Park *et al.* 2005), neurological diseases (Brouwer *et al.* 2009) and inflammatory diseases of the lung (asthma, chronic obstructive pulmonary disease, sarcoidosis) and other organs or systemic diseases (rheumatoid arthritis, psoriasis, pterygium) have been reported to be associated with MSI (Samara *et al.* 2006). These identified markers may play a significant role in medical diagnostic applications and/or personalized therapy and are used according to the invention as biomarker or STR biomarker.

**Loss of Heterozygosity (LoH)** is a cross chromosomal event that results in loss of an entire chromosomal biomarker (e. g. gene, STR or another genetic polymorphism) within one parental genome copy of a diploid cell. In case of wild type heterozygosity, the analysis results by a germline or somatic mutation in a homozygous signal. **Allelic imbalance (AI)** is seen in case of a mixture of DNA derived from wild type and somatic mutant cells.

As used herein, a **contamination control** or **patient traceability control** is at least one additional biomarker that is coamplified in the multiplex PCR to verify the origin and concordance of a sample with an individual patient. In addition, cross contamination between reactions wells can be monitored by the parallel processing of several samples. This can be achieved by the use of patient (individual) specific forensic barcodes like polymorphic STRs with tetra- or penta-nucleotide repeats (Becker *et al.* 2007a, Becker et al. 2007b), biallelic deletion-insertion polymorphisms (EP2271777B1) and/or gonosomal sex-specific DNA markers like AMELXY (Haas-Rochholz and Weiler 1997), Y-chromosomal STRs or Y-chromosomal functional genes (e. g. SRY, TSPY, DXYS156, STS; Butler and Li 2014) which are co-amplified by multiplex-PCR, analyzed and deposited in an external external data processing system. Alternatively, the individual samples can be spiked with artificial DNA barcodes as control target sequences.

As used herein, the term **amplification/amplifying** (or duplication reaction) refers to a reaction that generates at least one copy of a particular polynucleotide sequence or increasing the copy number or amount of a particular polynucleotide sequence. The products of the amplification are also referred to as **amplicons** (=amplification product). For example, polynucleotide amplification may be a process using a polymerase, a pair of oligonucleotide primers and a buffer with a defined pH and co-substrates of the polymerase (e. g. NTPs or dNTPs, MgCl₂, monovalent cations like K⁺ or NH₄⁺, PCR enhancers like N,N,N-trimethylglycine, dimethyl sulfoxide, protein and nucleic acid stabilizers like bovine serum albumin, polyoxyethylene (20) sorbitan monolaurate, trehalose, 1,4,5,6-tetrahydro-2-methyl-4-pyrimidinecarboxylic acid, and biocides like sodium azide) for producing any particular polynucleotide sequence. Amplification may be accomplished by the *in vitro* methods of the polymerase chain reaction (PCR) which is well known to expert in this field of invention as state of the art.

**Multiplex** describes amplification (or duplication) and determination of multiple target nucleic acid sequences in one reaction setup. Multiplex means at least two, preferably four or more, six or more, eight or more microsatellites which are analyzed at the same time in the same reaction setup with the corresponding primer mixture suitable to amplify specifically all presented target sequences (specific for each microsatellite (STR Biomarker). The genetic fingerprint of human (forensic DNA profiling and paternity testing) by genotyping at least six or more, preferably 12, microsatellites (short tandem repeats), differential diagnostics of different somatic mutations in tumors, clarification of organ-specific infections (e. g. lung, intestine, sexually transmitted infections) by detection of specific pathogen groups and/or amplification of nucleic acid libraries (panels) are examples for multiplex methods. Preferably, the method according to the invention is a multiplex method which may be used for any desired type - equal or analogous to the stated examples - of detection. In a preferred embodiment the method for diagnosis is designed and validated as multiplex.

**Modaplex detection system or Modaplex device** (former also published as ICEplex or STAR technology; EP1601780B1; Hlousek *et al.* 2012) is an automated high multiplex real time qPCR platform technology which combines within an integrated system solution a PCR thermocycler and a capillary electrophoreses (CE) device (Biotype GmbH, Dresden, DE). The fluorescence detection unit of the CE comprises at least one, preferably at least two, fluorescence excitation source (gas laser, diode laser, light-emitting diode) and at least one appropriate fluorescence detection channel. The current optical configuration of the Modaplex system consists of a blue argon laser (excitation 488 nm) and a red light-emitting diode (excitation maximum 637 nm, band-pass ± 6.5 nm) in combination with two fluorescence emission channels of 500-620 nm and >644 nm to detect relative fluorescence units (RFUs) of the dyes 6-carboxyfluorecein (6-FAM) and 5'-TYE™ 665 (proprietary of Integrated DNA Technologies Inc., San Diego, CA-US), respectively. Labeled DNA amplicons are electrokinetically injected at least 11fold into the CE with intervals of two PCR thermocycles and quantified giving rise to an amplification plot which can be used for nucleic acid quantification. The CE offers the separation of multiplex amplification products of at least up to 50 biomarkers or target nucleic acid sequences in a single reaction tube. The calculated separating capacity of the CE is at least ≥5 -10 bp, although secondary structures of the DNA amplicons may further influence their apparent motility during electrophoresis which must be considered for multiplex-PCR design.

There are currently also other **capillary electrophoresis instruments** beside the Modaplex system available which can support the present invention, although only in combination with a separate thermocycler device and only for qualitative endpoint detection. Examples of these instruments are ThermoFisher Scientific Inc. (Applied Biosystems Division, Carlsbad, CA-US) ABI Prism® Genetic Analyzer series instruments (e.g. 310, 3100, 3500), Promega Inc. (Fitchburg, WI-US) Spectrum and Spectrum Compact CE System, and Agilent Technologies Inc. (Santa Clara, CA-US) 4200 TapeStation System, Agilent AATI Fragment Analyzer and Agilent 2100 Bioanalyzer instrument. Other instruments are not excluded. However in these cases, primer design for multiplex applications, detection labels and calibrators must be adapted to the technical capability characteristics of these instruments (detection channels, separation capability of the electrophoresis).

As used herein, **Modaplex calibrators** are at least one, preferably two or more coamplified PCR products detected in capillary electrophoresis in at least one fluorescence channel in a multiplex setup, which are unrelated to the analytes or biomarkers of interest (unrelated or artificial templates and primers). They define the range of amplicon detection by the analyzing software of the Modaplex instrument. In the described example of this invention **Modaplex calibrator Mix 2** (Biotype GmbH, Dresden, DE) was used in a 1fold final concentration. It consists of artificial templates (unrelated to human DNA) and the corresponding PCR primer pairs. In the 6-FAM and 5'-TYE™ 665 fluorescence channel, respectively, three calibrators of different size were amplified. The calculated amplicon sizes were 110 bp, 249 bp, 306 bp. However, the apparent length can slightly differ by a maximum of 5 bp without impact on assay analysis. The Modaplex calibrators also represent a template-independent PCR control and should be added to all sample, negative and positive control wells. Alternatively, the PCR setup can be spiked by calibrators in an appropriate endpoint concentration. In this case other markers must be used as a template-independent PCR control. The regions between the Modaplex calibrators can be subdivided into regions for target, allele or profile calling of the amplicons of interest. However, an exact amplicon size calling is not possible.

As used herein, a **primer** refers to an oligonucleotide molecule (i. e., DNA or RNA) capable of annealing to a polynucleotide template (selected from target or wild type sequences) and provides a 3'-OH-end to produce a nucleic acid dependent DNA polymerase extension product which is complementary to the polynucleotide template. In case of a singleplex amplification reaction a pair of oligonucleotide primers which is flanking the desired nucleic acid region, in particular of the target sequence and of the wildtype sequence, and which is annealing to the forward and reverse polynucleotide strand, respectively, is used to generate a specific amplicon. In case of a multiplex reaction a plurality of primer pairs is used in one reaction setup. The primers may consist of natural NTPs (ribonucleotide triphosphates) or dNTPs (deoxyribonucleoside triphosphates). However, a variety of chemical modifications which modulating especially the annealing temperatures of the primers and the specificity of the amplification reaction are known to experts skilled in the art, especially for multiplex setups (concerning modifications of nucleobases, sugar ring and backbone linkage of the nucleotide; Bergstrom 2009, Malyshev and Romesberg 2015, Anosova *et al.* 2016, Houlihan *et al.* 2017 and references therein). To be detected by Modaplex capillary electrophoresis one primer of each primer pair must be covalently labeled with an instrument compatible fluorescent dye. Additionally, one primer of a primer pair may be modified at its 5'-end by an artificial tailing sequence to modulate the migration behavior in capillary electrophoresis for unambiguous resolution of amplicons in a multiplex reaction. These tails may be artificial nucleotide sequences, preferentially with high adenosine and/or thymidine content (AA, TT, TA, AT; which have less influence on the annealing temperature) or PCR stoppers like hexaethylene glycol moieties (US6756205B2 patent family) or e. g. twisted intercalating nucleic acid building blocks (Schneider *et al.* 2012) covalently linked alone or in combinations with further 5'-end nucleotide extensions or other chemical modifications.

As used herein, **label** or **detectable label** refers to any moiety or molecule which can be used to provide a detectable (preferably quantifiable) signal. A labeled nucleotide (e. g. a dNTP), or labeled oligo - (primer) or polynucleotide (amplicon), is one nucleotide linked to a detectable label. The term linked preferentially encompasses in case of the Modaplex system covalently bonded labels. Preferentially, one primer of a desired nucleic acid (target sequence, wild type sequence and/or contamination control) specific primer pair is covalently modified at its 5'-end with a fluorophore which is compatible to the detection unit of the Modplex system. Suitable fluorescent dyes include fluorochromes such as Cy5, Cy3, rhodamine and derivatives (such as Texas Red), 5'-TYE™ 665 (Integrated DNA Technologies Inc., San Diego, CA-US), fluorescein and derivatives (such as 6-carboxyfluorescein, 5-bromomethyl fluorescein), Lucifer Yellow, IAEDANS, 7-Me₂N-coumarin-4-acetate, 7-OH-4-CH₃-coumarin-3-acetate, 7-NH₂-4-CH₃-coumarin-3-acetate (AMCA), monobromobimane, pyrene trisulfonates, such as Cascade Blue, and monobromorimethyl-ammoniobimane (see for example, DeLuca, Immunofluorescence Analysis, in Antibody As a Tool, Marchalonis et al., eds., John Wiley & Sons, Ltd., 1982). For other instruments (Agilent Technologies Inc.) fluorescent dyes which selectively and non-covalently bind to double stranded DNA as intercalators or minor groove binders like SYBRGreen® (EP0512334B1) or EvaGreen® (Sang and Ren 2006) are used.

**Stutters** have been well characterized for PCR amplicon analysis in capillary electrophoresis (CE) technologies. They are generated especially during the amplification of STR regions by slipped-strand misalignment of *Taq* DNA polymerase (insertion or deletion of one or several repeat units according to replication slippage). The frequency of these artefacts is higher for STRs with repeat units of 1 - 3 bp. Thus, STRs with 4 or 5 bp repeats are standards for forensic applications to minimize the stutter problem concurrently keeping the amplicon size sufficiently small for forensic stain analysis (fragmented DNA). A further CE stutter artefact is caused by the intrinsic template independent nucleotidyl transferase activity of *Taq* DNA polymerase which produces 3'-adenine overhangs. The latter problem can be reduced to some extend by a prolonged final extension PCR thermocycling step (not possible for Modaplex technology due to the periodic injection into CE) and sequence specific artificial 5'-tailing of the non-labeled locus specific PCR primers.

A **profile or CE profile** as defined in this invention are DNA profiles of PCR amplicons which are not resolved to single molecule peaks within the CE electropherograms of the method according to the present invention, in particular according to the herein described Modaplex technology. In case of STRs DNA profiles comprise the sum of all data-pairs (RFUs over electrophoretic analyte retention times), of true STR alleles (up to 2 for heterozygous wild type DNA) and their corresponding stutter artefacts. The DNA profiles of altered STRs - target profile or target Modaplex profile - can be only interpreted and quantified in comparison to a wild type profile or wild type Modaplex profile as defined above (e. g. wild type nucleic acid in case of mutations).

Another aspect of the method according to the present invention it is an automated method for determining at least one MSI, wherein at least the steps of amplification, separation and detection, preferably amplification, separation, detection and calculation, are performed automatically in a closed system. Preferably, all steps are performed automatically and controlled and/or operated by software. More preferably the CE profiles and/or the resulting CE profile comparison are presented visually on an output medium automatically. Output medium comprise in digital data format a monitor and in analog format a printer.

The inventive method - also called MSI Modaplex method - is automatically controlled and operated by a computer program. Said program is saved on a processor (microchip or the like) within hardware. Said hardware is preferably part of the "MSI modaplex device" which combines the capillary electrophoresis components as well as all components of a computer and therefore performing as a fully closed system. Preferably, the MSI modaplex device fulfills all necessary regulations for a medical device for use in in-vitro-diagnostic.

In a preferred embodiment the inventive method is performed by means of the "MSI modaplex device" which comprises an interface for a digital data transmission (W-Lan, Bluethooth, network cable, USB) to and with an external "data source". "Data source" within the meaning of the present invention is another computer or storage medium within the same network or within another network of a third party. Third party comprises health organizations which generate, process and/or store patient-related data, like health insurance companies, hospitals, medical practices, research institutions, clinical centers, medical service providers and public authorities.

The other "data source" of a third party may provide additional data in context with the medical indication for which potential STR biomarker of interest are determined by the inventive method and preferably evaluated by the inventive method for diagnosis. Said medical indication comprises inflammation, cancer, inflammation associated cancer and/or auto immune diseases and are preferably are associated with at least one STR biomarker selected from mononucleotide, quasimonomorphic mononucleotide and/or dinucleotide repeats (see Table 2). The other "data source" of a third party can be a LIS (laboratory information system) and/or HIS (hospital information systems). Thus, the evaluation within the method for diagnosis take into account relevant data from a third party and calculate a therapy panel or recommendation based on the CE profile shift identified within the inventive method and based on additional data in context with general health data concerning the fitness data of the patient (heart activity, other risks), medical history as well as other physiological parameters and pathological parameters (results from imaging methods (e. g. ultrasonography, X-ray, positron emission tomography, etc., measures in clinical chemistry, other genotyping results, and therapeutic measures).

In one embodiment the whole method for determining and the method of diagnosis is controlled and operated by one computer program. In another embodiment the method for determining and the method of diagnosis are controlled and operated by two different and independent computer programs for which a data transfer from the Modaplex device to another computer can be necessary (delocalized calculation and comparison of the method).

In another aspect of the method according to the present invention the MS wild type sequence represents the unaffected microsatellite locus of interest and the MSI target sequence represents the microsatellite locus of interest which potentially is affected by a MSI due to a mutation in the mismatch repair system. In one preferred embodiment both sequences are obtained from the same source (patient, plant, animal), wherein the wild type sequence is from an unaffected region of the source (tissue, blood, mixture thereof) which is/should/can be located adjacent to the potentially affected region wherefrom the target sequence is isolated.

The above preferred embodiment, wherein both sequences are obtained from the same source (human, animal, plant) is not applicable wherein the source exhibits a genetic predisposition conferred by heredity. In such cases it could be of interest to use a digital wild type sequence or already saved and available digital wild type CE profile.

Therefore in another embodiment the wild type sequence is a digital sequence or already digital wild type CE profile which former was obtained from at least one unaffected region of a real source (tissue, blood, mixture thereof) and later was saved as a digital wild type CE profile. Preferably, said digital wild type CE profile is achieved from more than one original source and represents a statistically significant digital wild type CE profile or the specific MS locus of interest. Preferably, digital CE wild type profiles are applicable for mononucleotide and quasi monomorphic STR biomarker, wherein said digital CE wild type profile is based on a validated record of a significant cohort considering various wild type microsatellite sequences and their various respective allelic sequences.

Essentially the MS/MSI CE profile is a DNA profile of the achieved labeled amplicons which are not resolved to single molecule peaks within a CE electropherograms and preferably the respective CE profiles of the target and/or MS wild type sequence comprise the sum of peak areas of all true microsatellite alleles and corresponding stutter artefacts.]

In another aspect of the method according to the present invention the shift in the MSI target profile of at least one microsatellite of interest is an evidence for a medical indication which is associated with the at least one selected microsatellite. Thus, the comparison of both, the profile of the wild type sequence (being not affected or mutated) and the target sequence (potentially having a mutation) visualizes the relative difference in between said sequences, wherein the difference was caused by a mutation in the mismatch repair system in the cells, comprising in biological sample as defined above, wherefrom the target sequence was isolated. Thus, where a CE profile shift cannot be determined the target sequence potentially does not comprise any mutation. Alternatively, there is no difference between said profiles because the sequences (target vs. wild type) are identical due to a genetic predisposition conferred by heredity.

Preferably, the at least one selected microsatellite is a STR biomarker associated with and specific for an inflammation, cancer, inflammation associated cancer and/or auto immune disease. More preferably the specific STR biomarker is/are selected from at least one mononucleotide, quasimonomorphic mononucleotide and/or dinucleotide repeats (see Table 1 below).

**Table 1: Target Sequences of preferred MS loci (STR biomarker) according to the invention**

| **STR marker** | **Gen Bank® ID** | **Repeat unit** | **Seq-ID** | **Sequence** |
|---|---|---|---|---|
| NR-21 | XM_033393 | (T)₂₁ 5'UTR | 11 | XM_033393 (301 bp - 700bp, direct) 400bp |
| | | | | TCTCCTGTTTTATTAGGGGGAGAGGTGAAGGGAAATCCAGGCTCACTTTCTGAATAAGCCACTG CCTGGTGCACAGAGCAGAACCATCCTGGTTTCTGAAGACACATCCCTTTCAGCAGAATTCCAGC CGGAGTCGCTGGCACAGTTCTATTTTATATTTAAATGTATGTCTCCCCTGGCCTTTTTTTTTTTT TTTTTTTTTAGCAACACTTTTCTTGTTTGTAAACGCGAGTGACCAGAAAGTGTGAATGCGGAGTA GGAATATTTTTCGTGTTCTCTTTTATCTGCTTGCCTTTTTTAGAGAGTAGCAGTGGTTCCTATTTC GGAAAAGGACGTTCTAATTCAAAGCTCTCTCCCAATATATTTACACGAATACGCATTTAGAAAGG GAGGCAGCTT |
| NR-24 | X60152 | (T)₂₄ 3'UTR | 12 | X60152 (3001 bp - 3400bp, direct) 400bp |
| | | | | TGTTAAGCCAGTCTTTGCAAATGACCCCTTCCTGCCCATCACTGCCCTTCCTCAAGACCTAAAA TAGCTCCCTATTTAGTGAAAAATTATCTGAATATTTAAGGTCTGCCTTAACGTGATCCCCATTGC TGAATTTTACCTCCTGACTCCAAAAACTCTTCTCTTCCCTGGGCCCAGTCCTATTTTTTTTTTTTT TTTTTTTTTTTGTGAGACAGAGTCTCACTCTGTCACCCAGGTTGGAATGCAATGGCACAATCTCC GCTCACTGCAACGTCCGCCTCCCGGGTTCACGCCATTCTCCTGCCTCAGCCTCCCGAATAGCT GGGACTACAGGTCGCCGCCAACACGCCTGGCTAATTTTTTGTATTTTTAGTAGAGACGGGGTTT CACCGTGTTAGCC |
| Bat-25 | L04143 | (T)₂₄ intron 16 | 13 | L04143 HUMCKIT (6701 bp - 71 00bp, direct) 400bp |
| | | | | AGAAAAATCCTCTCTTCCTCACAGGCTCATACATAGAAAGAGATGTGACTCCCGCCATCATGGA GGATGACGAGTTGGCCCTAGACTTAGAAGACTTGCTGAGCTTTTCTTACCAGGTGGCAAAGGG CATGGCTTTCCTCGCCTCCAAGAATGTAAGTGGGAGTGATTCTCTAAAGAGTTTTGTGTTTTGTT TTTTTGATTTTTTTTTTTTTTTTTTTTTTTTTGAGAACAGAGCATTTTAGAGCCATAGTTAAAAGCA GAATGTCATTTAAAACAAAAGTATTGGATTTTTTATAATATAAGCAACACTATAGTATTAAAAAGT TAGTTTTCACTCTTTACAAGTTAAAATGAATTTAAATGGTTTTCTTTTCTCCTCCAACCTAATAGT GTATTCACA |
| Bat-26 | NG_007110 | (A)₂₆ intron 5 | 14 | NG_007110 (16101 bp - 16500bp, direct) 400bp |
| | | | | ATCCAGTGGTATAGAAATCTTCGATTTTTAAATTCTTAATTTTAGGTTGCAGTTTCATCACTGTCT |
| | | | | GCGGTAATCAAGTTTTTAGAACTCTTATCAGATGATTCCAACTTTGGACAGTTTGAACTGACTAC TTTTGACTTCAGCCAGTATATGAAATTGGATATTGCAGCAGTCAGAGCCCTTAACCTTTTTCAGG TAAAAAAAAAAAAAAAAAAAAAAAAAAAAGGGTTAAAAATGTTGAATGGTTAAAAAATGTTTTCATT GACATATACTGAAGAAGCTTATAAAGGAGCTAAAATATTTTGAAATATTATTATACTTGGATTAGA TAACTAGCTTTAAATGGCTGTATTTTTCTCTCCCCTCCTCCACTCCACTTTTTAACTTTTTTTTTTT TAAGT |
| Mono27 | AC007684 | (A)₂₇ | 15 | AC007684 (112701 bp - 113100bp, direct) 400bp |
| | | | | ATACCAGGCCACTGGGCAGCTGGCACATATTTATATAATTTATAGCTATTGGTATTTACCATATT AGAAATTAAAACAAATTTTTATATCAGCTGTATGCTTCAATTTCATTGTTATGTGAACCACCTATG AATTGCAGATCCTGGATGGCTTCAGATTTATTTTGGGCTTCACTCTTTTTTTTTTTTTTTTTTTTT TTTTTGAGACAGAGTCTAGCTCTGTCACTCAGGCTGGAGTGCAGTGGAACGATCTCTGCTCACT GCAAGCTCCGCCTCCCGGGTTCATGCCATTCTCCTGCCTCAGTCTCCCGAGTAGCTGGGACTA CAGGCACCCACCACCACGCCTGGCTAATTTCTTCTTGTATTTTTAGTAGAGACGTGGTTTCACC GTGTTAGCCAG |

According to the well-known "Bethesda panel" two mononucleotide (BAT-25 and BAT-26) and three dinucleotide (D2S123, D5S346, and D17S250) repeat microsatellites are recommended for the detection of MSI in colorectal cancer, and according to which tumors presenting two or more unstable markers (or ≥30-40% if more markers are tested) should be defined as MSI/MSI-H. According to the present invention the combination of D2S123, D5S346, D17S250, Bat-25 and/or Bat-26 NR-21, NR-24, Mono27 is proposed.

In another aspect of the method according to the present invention the MSI target sequence and it specific wild type are selected from the STR biomarker comprising NR-21, NR-22, NR-24, NR-27, D2S123, D5S346, D17S250, Mono27, CAT-25, HT-17, Bat-52, Bat-55, Bat-56, Bat-57, Bat-59, Bat-25 and/or Bat-26. The aforementioned biomarker are defined in table 1 and each is defined as a microsatellite locus: NR-21 as a 21T repeat located in the 5' untranslated region of the SLC7A8 gene; NR-24 as a 24T repeat located in the 3' untranslated region of the zinc finger-2 gene; NR-22 as a 22T repeat located in the 3' untranslated region of the putative transmembrane precursor protein B5 gene and/or NR-27 as a 27A repeat located in the 5' untranslated region of the inhibitor of apoptosis protein-1 gene.

For high MSI tumors a panel of five STR biomarker may be preferred; Bat-26, Bat-40, Mfdl5, APC and D2S123. APC and D2S123 show highest sensitivity for the recognition of both low and high MSI tumors. While Bat-26, Bat-40 and Mfdl5 exhibit the most specific behavior in detecting the high MSI tumors. D2S123, D5S346, D17S250, Bat-25 and/or Bat-26 NR-21, NR-24, Mono27. Loci with many clear shifts are known for almost all mononucleotide repeats (Bat-25, Bat-26, Bat-4O, 52C10, and SOCIO; Table 2), wherein the mononucleotide repeat sequences amplified by Bat-26 and Bat-4O show the clearest shifts.

In another aspect of the method according to the present invention the MSI target sequence and it specific MS wild type sequences are selected from the STR Biomarker NR-21, NR-24, Mono27, D2S123, D5S346, D17S250, Bat-25 and/or Bat-26. The inventive method is also applicable to new biomarker for another indication for which MSI is reported. The skilled person is able to achieve appropriate primer for new biomarker by applying state of the art primer design methods. MSI-H is defined as ≥ 30 -40%, MSI-L is defined as < 30-40% und MSS = 0%.

In another aspect of the method according to the present invention the at least one CE profile shift is associated with an inflammation, cancer, inflammation associated cancer and/or auto immune disease. *Examples are for* LOH and MSI in benign diseases, such as actinic keratosis, pterygium, atherosclerosis, asthma, chronic obstructive pulmonary disease, sarcoidosis and idiopathic pulmonary fibrosis. Examples are for LOH and MSI and chronical inflammatory diseases, such as Endometriosis, Leiomyoma, Adenomyosis, Chronical ulcerative colitis, Crohn's disease (less common), adenomatous polyps of the colon, chronic gastritis, Barrett's esophagus, Pancreatitis. Examples for ICI pathway and infection, such as Accute or chronical infections which can cause cancer, infections without cancer and sepsis.

Another application of the inventive method is directed to Pre-clinical studies, wherein cytotoxicity is analyzed based on MS loci and potential shifts in its MS CE profiles.

In another aspect of the method according to the present invention at least three, preferably at least four or more different microsatellites are analyzed simultaneously. A preferred embodiment defines a multiplex method for determining of at least one microsatellite instability (MSI) based on a shift in a capillary electrophoresis (CE) profile (CE profile shift = profile shift) comprising at least five, six, seven, eight, nine, 10, greater or equal to 11, 12, 13, 1, 20 or more microsatellites of respective different STR biomarker (table 1, 2). Following the Bethesda guidelines STR biomarker D2S123, D5S346, D17S250, Bat-25 and/or Bat-26 are analyzed. According to revised Bethesda guidelines the STR biomarker NR-21, NR-24, Mono27, Bat-25 and/or Bat-26. In a preferred embodiment eight different STR biomarker are analyzed, in particular selected from D2S123, D5S346, D17S250, Bat-25 and/or Bat-26 NR-21, NR-24, Mono27.

Another aspect of the present invention is an MSI target profile of at least one target sequence and/or an MS wild type profile of at least one wild type sequence achieved by the inventive method for determining of at least one microsatellite instability (MSI) based on a shift in a capillary CE profile. Preferably, the MSI target profile and/or the MS wild type profile are digital profiles, respectively, for a selected STR biomarker which was identified from a biological sample and for a patient.

Another aspect of the present invention the use of at least one primer pair specific for and suitable to amplify at least one target sequence of at least one microsatellite and its specific MS wild type sequence wherein at least one primer of the at least one primer pair comprise an artificial tailing sequence for the modulation of the migration behavior in CE and at least one primer of the primer pair comprise a fluorophore, wherein the artificial tailing sequence comprises a high adenosine and/or thymidine content (AA, TT, TA, AT) which have less influence on the annealing temperature, a hexa ethylene glycol moiety function as PCR stoppers and are also described in US6756205B2, or a twisted intercalating nucleic acid building block covalently linked alone or in combinations with further 5'-end nucleotide extensions (described in Schneider et al. 2012) or other chemical modifications.

The aforementioned primer and primer pairs are for use for the amplification of at least one MSI target and/or MS wild type sequence, which preferably are isolated from a biological sample, in the method, preferably multiplex method, for determining of at least one microsatellite instability (MSI) based on a shift in a capillary CE profile.

In another aspect of the use according to the present invention it is for achieving labeled amplicons of at least one MSI target sequence and of at least one MS wild type sequence which are detectable in the inventive method, preferably multiplex method, for determining of at least one microsatellite instability (MSI) based on a shift in a capillary CE profile as described above.

In another aspect of the use according to the present invention it is for in-vitro analysis of a nucleic acids comprising in a biological sample for the determination of at least one microsatellite instability (MSI) based on a shift in a capillary CE profile as described above.

The inventive primer use enables the skilled person to achieve a MSI target profile and a MS wild type profile by means of the inventive method as described above. The primer pairs are created in that said primer allows distinct discrimination in the inventive method. Within the meaning of the present invention any primer pair specific for and suitable to amplify at least one STR biomarker as summarized in table 2 below can be created by use of state of the art method by any skilled person.

**Table 2: Definitions of representative MS loci for which the inventive method is applicable.**

| **STR Biomarker name** | **Gene / chromosomal location** | **GenBank© ID (where available)** | **Repeat unit** |
|---|---|---|---|
| NR-27 | inhibitor of apoptosis protein-1 | AF070674 | (A)₂₇ 5'UTR |
| Bat-25 | c-kit oncogene / 4q12 | U63834 / 98345808 | (A)ₙ |
| Bat-26 | hMSH2 / 2p | 9834505, U41210 | (A)ₙ in intron 5 |
| Bat-40 | 3-f3-hydroxy-steroid dehydro-genase / 1 p13.1 | M38180 | TTTT.TT..(T)₇[...] TTTT.(T)₄₀ |
| Bat-52/55/56/57/59 | | GRCh38 | (A)ₙ |
| | | | n= 52, 55, 56, 57, 59 |
| Mono-11 | | AC007684 | Mononucleotide |
| Mono-15 | | AC007684 | mononucleotide |
| CAT25 | CASP2 gene | | (T)₂₅ 3'UTR |
| HT-17 | Heat shock protein | | |
| PTHL3 | | | (A)₁₀ |
| SEC63 | | | (A)₁₀ |
| HPDMPK | | | (T)₁₄ |
| U79260 | | | (T)₁₄ |
| D5S346 (APC) | adenomatous polyosis coli gene / | 181171 | (CA)₂₆ |
| D17S250 | 17q11.2-BRCA1 | 177030 | Dinucleotide |
| Mfd15 (D17S261) | 17p12-11.1 | | (CA)₁₆ |
| D2S123 | 2p16 hMS H2 | 187953 | |

A further aspect of the present invention is a primer pair specific for and suitable to amplify at least one target sequence of at least one microsatellite locus (STR biomarker), in particular selected from table 1, applicable or applying in the method for determining of at least one microsatellite instability (MSI) based on a shift in a capillary electrophoresis (CE) profile (CE profile shift = profile shift) and preferably in the inventive method for diagnosis of a MSI phenotype associated with an inflammation, cancer, inflammation associated cancer and/or auto immune disease, wherein the inventive primer pairs are having the sequences selected from
- for NR-21 Seq ID No: 1 and Seq ID No: 2,
- for NR-24 Seq ID No: 3 and Seq ID No: 4,
- for Bat-25 Seq ID No: 5 and Seq ID No: 6,
- for Bat-26 Seq ID No: 7 and Seq ID No: 8, and/or
- for Mono27 Seq ID No: 9 and Seq ID No: 10 or any combination of said pairs.

**Table 3: Sequences of the primer pairs according to the invention (MSI multiplex PCR)**

| **Primer** | | |
|---|---|---|
| **Seq -ID** | **DNA Sequence (5' →3')** | **5'-Label** |
| 1 | CAGAA TTCCAGCCGGAGTCG | F |
| 2 | AAATTTTAAATCTGGTCACTCGCGTTTACA | |
| 3 | AATATTTTTATATTAAAAATATTTAAAA TATTAAATATCTGCCTTAACGTGATCCCC | T |
| 4 | TTTTAATTAATATAAAAATAATTTTAAAAA TTTAAATTTTAAAATTATTTTTTTAATAA ATTAATTCATTGCATTCCAACCTGGGTG | |
| 5 | AATTTTTTAAAATTTTATAAATAAATATT TAAAATTTAAATATTAATTTAGAATGT AAGTGGGAGTGATTCTC | T |
| 6 | ATTATTATATTATTATATTTTAAATTATA TTAATATTAAAAAATAAAAAAATATAAA ATTATATTTAATTAATAATATGGCTCTAAA ATGCTCTGTTC | |
| 7 | ATAAATGAAATTGGATATTGCAGCAGTCAG | |
| 8 | TTATTAATATTAAATTTTAAAGCTCCTTTATAAGCTTCTTCAG | F |
| 9 | AAAAATTAAAAATTAATAAATATTAATAAGATGGCTTCAGATTTATTTTGGG C | F |
| 10 | TATTTATATTATAAATTATTATTTATATAAATTAAATTTTAAATATAATATTGC AGGAGAATGGCATGAACC | |

The skilled person is able to create or design further appropriate primer pairs for us in the inventive methods and which are specific for and suitable to amply at least one STR biomarker of table 2 by applying state of the primer design methods.

A further aspect of the present invention is a Kit for analyzing, in particular determining, of at least one CE profile shift according to the method as defined above, wherein the CE profile shift being determined by a comparison between the capillary electrophoresis (CE) profile of a target sequence of at least one microsatellite (MSI target CE profile = target profile) and the capillary electrophoresis (CE) profile of its specific wild type sequence (MS wild type CE profile = wild type pofile), the kit, (also called "MSI Modaplex Analysis Kit") comprises
- at least one pair of primers specific for and suitable to amplify the at least one target sequence of at least one microsatellite to be analyzed, wherein each of the least one primer pair comprise an artificial tailing sequence and at least one primer of the primer pair comprise a fluorophore, as defined above
- at least one calibration sequence (synonym for calibrator as defined above),
- at least one pair of primers specific for and suitable to amplify the at least one calibration sequence, and a
- contamination control.

In another aspect of the present invention the kit comprises at least one pair of primer specific for and suitable to amplify at least one MS locus selected from NR-21, NR-24, Mono27, D2S123, D5S346, D17S250, Bat-25 and/or Bat-26. Preferably, the primer pairs of the kit according to the invention are having the Seq ID Nos for NR-21 Seq ID No: 1 and Seq ID No: 2, for NR-24 Seq ID No: 3 and Seq ID No: 4, for Bat-25 Seq ID No: 5 and Seq ID No: 6, for Bat-26 Seq ID No: 7 and Seq ID No: 8, and for Mono27 Seq ID No: 9 and Seq ID No: 10.

Further components which are necessary for performing a PCR are preferably part of the above kit and are selected from an appropriate Primer Mix (as defined above) a PCR buffer, enhancer, thermostable DNA Polymerase, an aliquot of the nucleic acid sequence of the at least one Modaplex calibrator, preferably at least one contamination control and nucleic acid free water. For a successful method according to the present invention from greater than or equal 0.5 to less than or equal to 5 ng nucleic acid are added to the reaction setup. Preferably, 2 ng nucleic acids.

The inventive kit comprise all necessary reagents for a successful MSI Modaplex analysis, preferably MSI in vitro diagnosis, at least for the STR biomarker Bat-25, Bat-26, NR-21, NR-24, Mono27, D2S123, D5S346 and/or D17S250. And is for use by means of the Modaplex device and described herein. Further the inventive kits enable the skilled person to achieve CE profiles by use of a suitable software, preferably the Moda-RA Software, which calculates the CE profiles of the present invention on the basis of the raw data achieved from amplifying, separating and detecting according to the inventive methods.

In another embodiment of the inventive kit as defined above it is designed for determining an MSI shift in a target sequence of at least one MS locus (STR biomarker), preferably associated with an inflammation, cancer, inflammation associated cancer and/or auto immune disease, as defined above.

A further aspect of the present invention is a primer pair, as defined above, or a kit, as defined above for us in an automated capillary electrophoresis (CE) performed by means of an automated CE device (preferably the automated Modaplex System), preferably for use in the herein described method for determining of at least one microsatellite instability (MSI) based on a shift in a capillary electrophoresis (CE) profile (CE profile shift = profile shift), the CE profile shift being determined by a comparison between the capillary electrophoresis (CE) profile of a target sequence of at least one microsatellite (MSI target CE profile = target profile) and the capillary electrophoresis (CE) profile of its specific wild type sequence (MS wild type CE profile = wild type profile) and in the method for diagnosis as described below.

Therefore, another aspect of the present invention is a method for, preferably a [automatically controlled and operated method by computer program, diagnosis of a MSI phenotype associated with an inflammation, cancer, inflammation associated cancer and/or auto immune disease, wherein said method comprises determining of at least one CE profile shift, preferably determining of at least one microsatellite instability (MSI) based on a shift in a capillary electrophoresis (CE) profile (CE profile shift = profile shift), the CE profile shift being determined by a comparison between the capillary electrophoresis (CE) profile of a target sequence of at least one microsatellite (MSI target CE profile = target profile) and the capillary electrophoresis (CE) profile of its specific wild type sequence (MS wild type CE profile = wild type profile), as described above.

In an embodiment of the method for diagnosis according to the invention, the further a step of calculation a MSI score representing the degree of the identified shift of the selected MS locus is comprised.

In another embodiment of the method for diagnosis according to the invention, the method comprising further a step of
- evaluation, in particular computer program based, of the identified CE profile shift as described above and/or of the calculated MSI score by considering further patient -related data associated with the health status of the patient at least at the time in which a biological sample was obtain from the patient and preferably the CE profile shift was identified (preferably data representing the progression of the health status), and
- generating, in particular automatically and computer program based, a diagnosis report considering and presenting all considered data (preferably presented visually on an output medium (monitor/print)), preferably said diagnosis report is generated digitally and/or is transmitted to the assigned physician or another recipient automatically via an interface to a respective network system and preferably
- presenting a tailored therapy plan recommendation for the prevention or treatment of an inflammation, cancer, inflammation associated cancer and/or auto immune disease, preferably which is part of the automatically generated diagnosis report.

For a valid diagnosis of any medical indication the variation range of different alleles of the same MS locus (STR biomarker) has to be considered. The same applies to patients (animal or human) exhibiting a genetic predisposition conferred by heredity. In such cases the MS wild type sequence and respective MS wild type profile is not achievable from the patient. Therefore, in such cases it could be of interest to use a digital wild type CE sequence or already saved and available digital wild type CE profile for the MS locus (STR biomarker) of interest.

Preferably, said digital wild type CE profile is achieved from more than one original source and represents a statistically significant digital wild type CE profile or the specific MS locus of interest. Preferably, digital CE wild type profiles are applicable for mononucleotide and quasi monomorphic STR biomarker, wherein said digital CE wild type profile is based on a validated record of a significant cohort considering various wild type microsatellite sequences and their various respective allelic sequences (Goel et al 2010).

A further aspect of the present invention is a device, in particular a "MSI Modaplex device" comprising at least one excitation source, which is variable but preferably selected from gas laser, diode laser, light-emitting diode, and least one detector for detecting the fluorophore labeled nucleic acids, in particular of the labeled amplicons obtained in the method as described above, at least one processor, a least one microchip and at least one interface for digital data transmission, wherein the processor comprise the at least one program controlling and operating the method for determining a MSI CE profile shift and/or the method for diagnosis of a MSI phenotype.

All necessary components of a CE are present in the above device and therefore presenting a complete and fully closed system. Preferably fulfilling all necessary regulations for a medical device for use in in-vitro-diagnostic.

In a preferred embodiment the inventive method for diagnosis is performed by means of the "MSI Modaplex device" which preferably comprises an interface for a digital data transmission (W-Lan, Bluethooth, network cable, USB) to and with an external "data source". "Data source" within the meaning of the present invention is another computer or storage medium within the same network or within another network of a third party. Third party comprises health organizations which generate, process and/or store patient-related data, like health insurance companies, hospitals, medical practices, research institutions, clinical centers, medical service providers and public authorities.

The other "data source" of a third party may provide additional data in context with the medical indication (Hereditary Non-Polyposis Colorectal Cancer (HNPCC), sporadic (13-28%) and hereditary non-polyposis (79%) CRC) for which potential STR biomarker of interest are determined by the inventive method and preferably evaluated by the inventive method for diagnosis. Said medical indication comprises inflammation, cancer, inflammation associated cancer and/or auto immune diseases and are preferably are associated with at least one STR biomarker selected from mononucleotide, quasimonomorphic mononucleotide and/or dinucleotide repeats (see Table 2).

The other "data source" of a third party can be a LIS (laboratory information system) and/or HIS (hospital information systems). Thus the evaluation within the method for diagnosis take into account relevant data from a third party and calculate a therapy panel based on the CE profile shift identified within the inventive method and based on additional data in context with general health data concerning the fitness data of the patient (heart activity, other risks), medical history as well as other physiological parameters and pathological parameters (results from imaging methods (e. g. ultrasonography, X-ray, positron emission tomography, etc., measures in clinical chemistry, other genotyping results, and therapeutic measures).

In a further embodiment of the diagnosis method, additionally the patient is monitored, wherein receiving with a processor communicatively connected to a data network nonmedical data corresponding to a patient from a social network service that is connected to the data network; identifying with the processor a health characteristic of the patient in the nonmedical data received from the social network service; generating with the processor a message including health advice associated with the identified health characteristic of the patient; and sending with the processor the message to an electronic device associated with the patient through the data network.

The "modaplex device" comprises an interface for a digital data transmission (W-Lan, Bluetooth, network, USB) to and with an external "data source". "Data source" within this meaning is another computer or storage medium within the same network or within another network of a third party. A third party could be a health organization which generates, processes and/or store patient-related data, comprising health insurance companies, hospitals, medical practices, research institutions, clinical centers, medical service providers and public authorities

The methods according to the present inventions are suitable for use in prediction of ICI efficacy therapy of (metastatic) tumors (Boland *et al.* 1998, current clinical guidelines), for determination of MSI and LoH of non-cancer diseases (Samara *et al.* 2006), for determination of preclinical toxicity and mutagenicity testing with cell cultures or laboratory animals (Chen *et al.* 2000, Beal *et al.* 2015) and for analysis in epidemiology of infectious agents (Chen *et al.* 2011) analyzing strain specific pathogen profiles.

### Example

Matched pairs (disease and normal) of formalin-fixed, paraffine-embedded (FFPE) tissue intersections which were pre-characterized as colon cancer positive were obtained from Indivumed GmbH (Hamburg, DE). Written informed consent of the patients were given. DNA was isolated with the QIAamp® DNA FFPE Tissue kit (Qiagen GmbH, Hilden, DE). The quality, amount and concentration of DNA was determined with a Qubit™ 3.0 Fluorometer (ThermoFisher Scientific, Waltham, MA-US) calibrated with standard DNA of the manufacturer. A pentaplex PCR was set up which comprises the five quasi-monomorphic mononucleotide STR-markers NR-21, NR-24, BAT-25, BAT-26 and Mono-27 as proposed by Bacher *et al.* (2004) which support the Revised Bethesda Guidelines for MSI diagnosis of gastrointestinal tumors (Umar *et al.* 2004). The primers used in the Modaplex multiplex assay are depicted in table 3 and within the sequence listing. The Revised Bethesda Guidelines define samples as MSS (microsatellite stabile), MSI-L (instable low) and MSI-H (instable high) if 0, 1-2 and 3-5 or more of quasi-monomorphic mononucleotide STR-markers are visually interpreted as affected, respectively.

The Multiplex-PCR consisted of a total volume of 25 µl with one fold **Modaplex buffer 3** (containing Tris/HCI buffer pH 8.0, 250 mM dNTPs, monovalent cations, enhancers and stabilizers; Biotype GmbH, Dresden, DE), 4 mM MgCl₂, 1 unit Modaplex *Taq* DNA polymerase T (Biotype GmbH, Dresden, DE), 0.5 - 1.0 µM of each primer pair and 2 ng genomic DNA. All reactions, samples, negative and positive controls, further included **Modaplex calibrator mix 2** in 1fold final concentration.

The PCR was performed within the Modaplex detection system without a lid and without lid-heating to allow periodical injection into the capillary electrophoresis. Therefore, the reaction wells were finally overlaid with a drop of mineral oil to avoid evaporation.

The automated Modaplex detection system was setup according to the instructions of the manufacturer. A three step (for 40 s each) thermocycling protocol was used with a DNA denaturation step of 96 - 98 °C, a DNA annealing step of 54 - 64 °C and a DNA extension step of 72°C. During PCR, amplification products were injected electrokinetically and separated in an automated capillary electrophoresis. Electrophoresis was performed periodically every second cycle at the end of the extension step, starting from PCR cycle 19 and ending with PCR cycle 41. Raw data were collected and imported into the **Modaplex Result Analyzer (Moda-RA) software** for DNA profile display which was then used for visual analysis as recommended by clinical guidelines for MSI analysis. The latter software can automatically superpose the amplicon profiles of the matched pairs of the patient samples. In Fig. 1 and 2 DNA profiles of the STR amplicons at the end of PCR cycle 39 are depicted in doted lines for a normal tissue (wild type sequence) and in through lines for the matched disease tissue (target sequence). It is important to emphasize that the plotted profiles of the Fig. 1A-B and 2A-B does not represent the real sizes or length of the respective amplicon. The definition of the x-axis [bp] is calculated based on the calibrators as defined above and could also been expressed as percent in respect to the calibrators or as retention time. Consequently, only relative sizes of each amplicon can be estimated compared to the calibrators.

Depending on the signal strength the raw data of other PCR cycles can be used for an overview or the presentation of individual biomarkers. The software allows to get at a first glance an overview of the multiplex PCR results (Fig. 1, A and B). The STR specific detection regions can then be individually enlarged by a computer mouse click. In doing so the relative fluorescent units (RFU, y axis) of the biomarker profiles are normalized to get comparable presentations for weakly and strongly amplified STRs (see Fig. 2, A and B). This facilitates the visual data analysis according to the current clinical guidelines.

As shown, the patient sample could be clearly interpreted as MSI-H (high MSI, Ulmar *et al.* 2004) because 5 out of the 5 markers could be analyzed to be affected by MSI.

### Figure legends

**Figure 1****: Electropherogram of the 6-FAM (****Fig. 1A****) labeled and of the 5'-TYE™ 665 (****Fig. 1B****) labeled biomarkers of the MSI multiplex PCR.**
   Raw data of matched pairs (disease tissue in through and normal tissue in doted lines) of FFPE tissue intersections were superposed with Modaplex Result Analyzer (Moda-RA) software and visualized as DNA profiles. The ranges which were used for the detection of the biomarkers are depicted as rectangles and labeled by the biomarker name. C1 and C2 are the profiles of calibrator 1 and 2 of the Modaplex calibrator Mix 2.
**Figure 2****: Enlarged electropherograms of the 6-FAM labeled biomarkers NR-21 and Mono-27 of the MSI multiplex PCR.** Raw data of matched pairs (disease tissue in through and normal tissue in doted lines) of FFPE tissue intersections were superposed with Modaplex Result Analyzer (Moda-RA) software, visualized as DNA profiles and individually enlarged by a computer mouse click. The RFU scale of the week MONO-27 signal (**Fig. 2B****,** compare to figure 1) was adjusted to NR-21 (**Fig. 2A****,** compare to figure 1) to facilitate the visual data analysis according to the current clinical guidelines. The ranges which were used for the detection of the biomarkers are depicted as rectangles and labeled by the biomarker name.

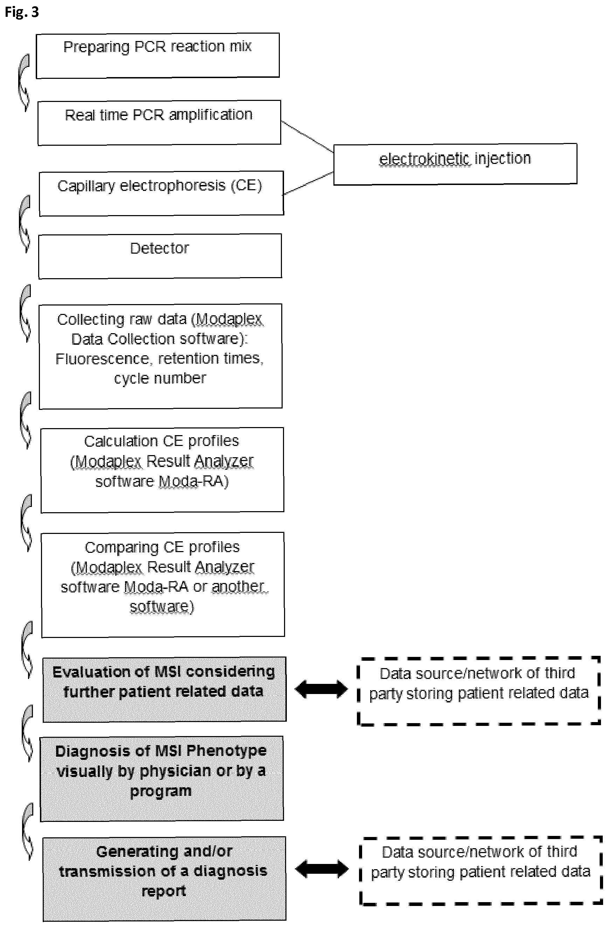

**Figure 3****: flow sheet of one embodiment of the inventive methods:** the steps according to claim 1 and according to claim 17 are summarized showing that according to the invention the methods are applicable in one passage without interruption in case of a complete validated in-vitro-diagnostic system and/or device.

### References:

Anosova I, Kowal EA, Dunn MR, et al. (2016). The structural diversity of artificial genetic polymers. Nucleic Acids Res 44: 1007-21.
Bacher JW, Flanagan LA, Smalley RL, et al. (2004). Development of a fluorescent multiplex assay for detection of MSI-High tumors. Dis Markers 20: 237-50.
Beal MA, Rowan-Carroll A, Campbell C, et al. (2015). Single-molecule PCR analysis of an unstable microsatellite for detecting mutations in sperm of mice exposed to chemical mutagens. Mutat Res 775: 26-32.
Becker D, Bender K, Edelmann J, et al. (2007a). New alleles and mutational events at 14 STR loci from different German populations. Forensic Sci Int Genet 1: 232-7.
Becker D, Vogelsang D, Brabetz W (2007b). Population data on the seven short tandem repeat loci D4S2366, D6S474, D14S608, D19S246, D20S480, D21S226 and D22S689 in a German population. Int J Legal Med 121: 78-81.
Bergstrom DE (2009). Unnatural nucleosides with unusual base pairing properties. Curr Protoc Nucleic Acid Chem Chapter 1: Unit 1.4.
Boland CR, Goel A (2010). Microsatellite instability in colorectal cancer. Gastroenterology 138: 2073-2087.
Boland CR, Thibodeau SN, Hamilton SR, et al. (1998). A National Cancer Institute Workshop on Microsatellite Instability for cancer detection and familial predisposition: development of international criteria for the determination of microsatellite instability in colorectal cancer. Cancer Res 58: 5248-57.
Bonneville R, Krook MA, Kautto EA, et al. (2017). Landscape of Microsatellite Instability Across 39 Cancer Types. JCO Precis Oncol doi: 10.1200/PO.17.00073 Epub 2017 Oct 3. PubMed PMID: 29850653.
Brouwer JR, Willemsen R, Oostra BA (2009). Microsatellite repeat instability and neurological disease. Bioessays 31: 71-83.
Butler E, Li R (2014). Genetic markers for sex identification in forensic DNA analysis. J Forensic Investigation 2(3): 10.
Chen M, Tan Z, Zeng (2011). Microsatellite is an important component of complete hepatitis C virus genomes. Infect Genet Evol 11: 1646-54.
Chen WD, Eshleman JR, Aminoshariae MR, et al. (2000). Cytotoxicity and mutagenicity of frameshift-inducing agent ICR191 in mismatch repair-deficient colon cancer cells. J Natl Cancer Inst 92: 480-5.
Colle R, Cohen R, Cochereau D, et al. (2017). Immunotherapy and patients treated for cancer with microsatellite instability. Bull Cancer 104: 42-51.
Goel Ajay, Takeshi Nagasaka, et al. (2010). An Optimized Pentaplex PCR for Detecting DNA Mismatch Repair-Deficient Colorectal Cancers. PLoSONE 5(2): e9393.
Haas-Rochholz H, Weiler G (1997). Additional primer sets for an amelogenin gene PCR-based DNA-sex test. Int J Legal Med 110: 312-5.
Hlousek L, Voronov S, Diankov V, et al. (2012). Automated high multiplex qPCR platform for simultaneous detection and quantification of multiple nucleic acid targets. Biotechniques 52: 316-24.
Houlihan G, Arangundy-Franklin S, Holliger P (2017). Exploring the Chemistry of Genetic Information Storage and Propagation through Polymerase Engineering. Acc Chem Res 50: 1079-1087.
Klaassen CH (2009). MLST versus microsatellites for typing Aspergillus fumigatus isolates. Med Mycol 47 Suppl 1: S27-33.
Malyshev DA, Romesberg FE (2015). The expanded genetic alphabet. Angew Chem Int Ed Engl 54: 11930-44.
Merritt BJ, Culley TM, Avanesyan A, et al. (2015). An empirical review: Characteristics of plant microsatellite markers that confer higher levels of genetic variation. Appl Plant Sci 3. pii: apps.1500025.
Miah G, Rafii MY, Ismail MR, et al. (2013). A review of microsatellite markers and their applications in rice breeding programs to improve blast disease resistance. Int J Mol Sci 14: 22499-528.
Park DI, Park SH, Kim SH, et al. (2005). Effect of Helicobacter pylori infection on the expression of DNA mismatch repair protein. Helicobacter 10: 179-84.
Romsos EL, Vallone PM (2015). Rapid PCR of STR markers: Applications to human identification. Forensic Sci Int Genet 18: 90-9.
Samara K, Zervou M, Siafakas NM, et al. (2006). Microsatellite DNA instability in benign lung diseases. Respir Med 100: 202-11.
Sang F, Ren J (2006). Capillary electrophoresis of double-stranded DNA fragments using a new fluorescence intercalating dye EvaGreen. J Sep Sci 29: 1275-80.
Schlötterer C (2000). Evolutionary dynamics of microsatellite DNA. Chromosoma 109: 365-71. Schneider UV, Mikkelsen ND, Lindqvist A, et al. (2012). Improved Efficiency and Robustness in qPCR and Multiplex End-Point PCR by Twisted Intercalating Nucleic Acid Modified Primers. PLoS ONE 7: e38451.
Svrcek M, Lascols O, Cohen R, et al. (2019). MSI/MMR-deficient tumor diagnosis: Which standard for screening and for diagnosis? Diagnostic modalities for the colon and other sites: Differences between tumors. Bull Cancer 106: 119-128.
Thiede C, Bornhäuser M, Ehninger G (2004). Evaluation of STR informativity for chimerism testing-comparative analysis of 27 STR systems in 203 matched related donor recipient pairs. Leukemia 18: 248-54.
Umar A, Boland CR, Terdiman JP, et al. (2004). Revised Bethesda Guidelines for hereditary nonpolyposis colorectal cancer (Lynch syndrome) and microsatellite instability. J Natl Cancer Inst 96: 261-8.
Vieira ML, Santini L, Diniz AL, Munhoz Cde F (2016). Microsatellite markers: what they mean and why they are so useful. Genet Mol Biol 39: 312-28.
Zhao J, Li T, Zhu C, et al. (2018). Selection and use of microsatellite markers for individual identification and meat traceability of six swine breeds in the Chinese market. Food Sci Technol Int 24: 292-300.

SeqIDs_multi-fasta.txt
>SeqID1, NR-21, artifical sequence, primer
CAGAATTCCAGCCGGAGTCG

>SeqID2, NR-21, artifical sequence, primer
AAATTTTAAATCTGGTCACTCGCGTTTACA

>SeqID3, NR-24, artifical sequence, primer
AATATTTTTATATTAAAAATATTTAAAATATTAAATATCTGCCTTAACGTGATCCCC

>SeqID4, NR-24, artifical sequence, primer
TTTTAATTAATATAAAAATAATTTTAAAAATTTAAATTTTAAAATTATTTTTTTAATAAATTAATTCATTGCATT
CCAACCTGGGTG

>SeqID5, BAT-25, artifical sequence, primer
AATTTTTTAAAATTTTATAAATAAATATTTAAAATTTAAATATTAATTTAGAATGTAAGTGGGAGTGATTCTC

>SeqID6, BAT-25, artifical sequence, primer
ATTATTATATTATTATATTTTAAATTATATTAATATTAAAAAATAAAAAAATATAAAATTATATTTAATTAATAA
TATGGCTCTAAAATGCTCTGTTC

>SeqID7, BAT-26, artifical sequence, primer
ATAAATGAAATTGGATATTGCAGCAGTCAG

>SeqID8, BAT-26, artifical sequence, primer
TTATTAATATTAAATTTTAAAGCTCCTTTATAAGCTTCTTCAG

>SeqID9, MONO-27, artifical sequence, primer
AAAAATTAAAAATTAATAAATATTAATAAGATGGCTTCAGATTTATTTTGGGC

>SeqID10, MONO-27, artifical sequence, primer
TATTTATATTATAAATTATTATTTATATAAATTAAATTTTAAATATAATATTGCAGGAGAATGGCATGAACC

>SeqID11, NR-21, XM_033393 (301bp - 700bp, direct) 400bp
TCTCCTGTTTTATTAGGGGGAGAGGTGAAGGGAAATCCAGGCTCACTTTCTGAATAAGCCACTGCCTGGT
GCACAGAGCAGAACCATCCTGGTTTCTGAAGACACATCCCTTTCAGCAGAATTCCAGCCGGAGTCGCTGG
CACAGTTCTATTTTTATATTTAAATGTATGTCTCCCCTGGCCTTTTTTTTTTTTTTTTTTTTTAGCAACA
CTTTTCTTGTTTGTAAACGCGAGTGACCAGAAAGTGTGAATGCGGAGTAGGAATATTTTTCGTGTTCTCT
TTTATCTGCTTGCCTTTTTTAGAGAGTAGCAGTGGTTCCTATTTCGGAAAAGGACGTTCTAATTCAAAGC
TCTCTCCCAATATATTTACACGAATACGCATTTAGAAAGGGAGGCAGCTT

>SeqID12, NR-24, X60152 (3001bp - 3400bp, direct) 400bp
TGTTAAGCCAGTCTTTGCAAATGACCCCTTCCTGCCCATCACTGCCCTTCCTCAAGACCTAAAATAGCTC
CCTATTTAGTGAAAAATTATCTGAATATTTAAGGTCTGCCTTAACGTGATCCCCATTGCTGAATTTTACC
TCCTGACTCCAAAAACTCTTCTCTTCCCTGGGCCCAGTCCTATTTTTTTTTTTTTTTTTTTTTTTTTGTGA
GACAGAGTCTCACTCTGTCACCCAGGTTGGAATGCAATGGCACAATCTCCGCTCACTGCAACGTCCGCCT
CCCGGGTTCACGCCATTCTCCTGCCTCAGCCTCCCGAATAGCTGGGACTACAGGTCGCCGCCAACACGCC
TGGCTAATTTTTTGTATTTTTAGTAGAGACGGGGTTTCACCGTGTTAGCC

>SeqID13, BAT-25, L04143 HUMCKIT (6701bp - 7100bp, direct) 400bp
AGAAAAATCCTCTCTTCCTCACAGGCTCATACATAGAAAGAGATGTGACTCCCGCCATCATGGAGGATGA
CGAGTTGGCCCTAGACTTAGAAGACTTGCTGAGCTTTTCTTACCAGGTGGCAAAGGGCATGGCTTTCCTC
GCCTCCAAGAATGTAAGTGGGAGTGATTCTCTAAAGAGTTTTGTGTTTTGTTTTTTTGATTTTTTTTTTT
TTTTTTTTTTTTTTGAGAACAGAGCATTTTAGAGCCATAGTTAAAAGCAGAATGTCATTTAAAACAAAAG
TATTGGATTTTTTATAATATAAGCAACACTATAGTATTAAAAAGTTAGTTTTCACTCTTTACAAGTTAAA
ATGAATTTAAATGGTTTTCTTTTCTCCTCCAACCTAATAGTGTATTCACA

>SeqID14, BAT-26, NG_007110 (16101bp - 16500bp, direct) 400bp
ATCCAGTGGTATAGAAATCTTCGATTTTTAAATTCTTAATTTTAGGTTGCAGTTTCATCACTGTCTGCGG
TAATCAAGTTTTTAGAACTCTTATCAGATGATTCCAACTTTGGACAGTTTGAACTGACTACTTTTGACTT
CAGCCAGTATATGAAATTGGATATTGCAGCAGTCAGAGCCCTTAACCTTTTTCAGGTAAAAAAAAAAAAA
AAAAAAAAAAAAAAGGGTTAAAAATGTTGAATGGTTAAAAAATGTTTTCATTGACATATACTGAAGAAGC
TTATAAAGGAGCTAAAATATTTTGAAATATTATTATACTTGGATTAGATAACTAGCTTTAAATGGCTGTA
TTTTTCTCTCCCCTCCTCCACTCCACTTTTTAACTTTTTTTTTTTTTAAGT

>SeqID15, MONO-27, AC007684 (112701bp - 113100bp, direct) 400bp

SeqIDs_multi-fasta.txt
ATACCAGGCCACTGGGCAGCTGGCACATATTTATATAATTTATAGCTATTGGTATTTACCATATTAGAAA
TTAAAACAAATTTTTATATCAGCTGTATGCTTCAATTTCATTGTTATGTGAACCACCTATGAATTGCAGA
TCCTGGATGGCTTCAGATTTATTTTGGGCTTCACTCTTTTTTTTTTTTTTTTTTTTTTTTTTTGAGACAG
AGTCTAGCTCTGTCACTCAGGCTGGAGTGCAGTGGAACGATCTCTGCTCACTGCAAGCTCCGCCTCCCGG
GTTCATGCCATTCTCCTGCCTCAGTCTCCCGAGTAGCTGGGACTACAGGCACCCACCACCACGCCTGGCT
AATTTCTTCTTGTATTTTTAGTAGAGACGTGGTTTCACCGTGTTAGCCAG

## Claims

1. A method for determining of at least one microsatellite instability (MSI) based on a shift in a capillary electrophoresis (CE) profile (CE profile shift), the CE profile shift being determined by a comparison between the capillary electrophoresis (CE) profile of a target sequence of at least one microsatellite (MSI target profile) and the capillary electrophoresis (CE) profile of its specific wild type sequence (MS wild type profile), the method comprises the steps of
- amplification by means of a real time PCR simultaneously of at least one target sequence and of at least one specific wild type sequence and labeling nucleic acids thereof during amplification,
- separating the labeled nucleic acids by capillary electrophoresis (CE) and detecting at least one fluorophore labeled amplicon of at least one target sequence and of at least one wild type sequence, respectively, in real time while amplification,
- computer program-based calculation of the at least one target profile and of the at least one corresponding wild type profile,
- comparing the at least one target profile and the corresponding wild type profile and
- determining a CE profile shift between the target profile and the corresponding wild type profile.

2. The method according to claim 1, wherein at least the steps of amplification, separation, detection and calculation is performed automatically in a closed system.

3. The method according to claim 1 or 2, wherein the MS wild type sequence represents the unaffected microsatellite locus of interest and the MSI target sequence represents the microsatellite locus of interest which potentially is affected by an MSI.

4. The method according to any one of the claims 1 to 3, wherein the shift in the MSI target profile of at least one microsatellite of interest is an evidence for a medical indication which is associated with the at least one selected microsatellite.

5. The method according to any one of the claims 1 to 4, wherein the MSI target sequence and its specific wild type sequence are selected from STR biomarker comprising NR-21, NR-22, NR-24, NR-27, D2S123, D5S346, D17S250, Mono27, CAT-25, HT-17, Bat-52, Bat-55, Bat-56, Bat-57, Bat-59, Bat-25 and/or Bat-26.

6. The method according to any one of the claims 1 to 5, wherein the MSI target sequence and its specific wild type sequences are selected from NR-21, NR-24, Mono27, D2S123, D5S346, D17S250, Bat-25 and/or Bat-26.

7. The method according to any one of the claims 1 to 6, wherein at least one CE profile shift is associated with an inflammation, cancer, inflammation associated cancer and/or auto immune disease.

8. The method according to any one of the claims 1 to 7, wherein at least four or more different microsatellites are analyzed simultaneously.

9. An MSI target profile of at least one target sequence and/or an MS wild type profile of at least one wild type sequence achieved by the method according to any one of the claims 1 to 8.

10. The use of at least one primer pair specific for and suitable to amplify at least one target sequence of at least one microsatellite and its specific wild type sequence wherein at least one primer of the at least one primer pair comprise an artificial tailing sequence for the modulation of the migration behavior in CE and at least one primer of the primer pair comprises a fluorophore, wherein the artificial tailing sequence comprises a high adenosine and/or thymidine content, a hexaethylene glycol moiety or a twisted intercalating nucleic acid building block covalently linked alone or in combinations with further 5'-end nucleotide extensions.

11. Use according to claim 10 to achieve labeled amplicons of at least one MSI target sequence and of at least one MS wild type sequence which are detectable in the method according to any one of the claims 1 to 8.

12. Use according to claim 10 or 11 for in-vitro analysis of nucleic acids comprising in a biological sample for the determination of at least one microsatellite instability (MSI) based on a shift in a capillary electrophoresis (CE) profile according to any one of the claims 1 to 8.

13. A primer pair specific for and suitable to amplify at least one MSI target sequence of at least one microsatellite locus applying in the method according to claim1 to 8, having the sequences
- for NR-21 Seq ID No: 1 and Seq ID No: 2
- for NR-24 Seq ID No: 3 and Seq ID No: 4
- for Bat-25 Seq ID No: 5 and Seq ID No: 6
- for Bat-26 Seq ID No: 7 and Seq ID No: 8
- for Mono27 Seq ID No: 9 and Seq ID No: 10

14. A Kit for analyzing of at least one CE profile shift comprising
- at least one pair of primers specific for and suitable to amplify the at least one target sequence of at least one microsatellite to be analyzed, wherein each of the least one primer pair comprise an artificial tailing sequence and at least one primer of the primer pair comprise a fluorophore,
- at least one calibration sequence,
- at least one pair of primers specific for and suitable to amplify the at least one calibration sequence, and a
- contamination control.

15. A Kit according to claim 14, wherein it comprises at least one pair of primer specific for and suitable to amplify at least one MS locus selected from NR-21, N-R24, Mono27, D2S123, D5S346, D17S250, Bat-25 and/or Bat-26.

16. A primer pair according to claim 10 to 13 or a kit according to any of the claims 14 or 15 for us in an automated capillary electrophoresis (CE) performed by means of an automated CE device.

17. A method for diagnosis of an MSI phenotype associated with an inflammation, cancer, inflammation associated cancer and/or auto immune disease, wherein said method comprises determining of at least one CE profile shift according to any one of the claims 1 to 8.

18. The method according to claim 17, comprising further a step of
- calculation of an MSI score representing the degree of the identified shift of the selected MS locus.

19. The method according to claim 17 or 18, comprising further a step of
- evaluation of the identified CE profile shift and/or of the calculated MSI score by considering further patient-related data associated with the health status of the patient at least at the time in which a biological sample was obtain from the patient and preferably the CE profile shift was identified, and
- generating a diagnosis report considering and presenting all considered data and preferably
- presenting a tailored therapy plan recommendation for the prevention or treatment of an inflammation, cancer, inflammation associated cancer and/or auto immune disease.

20. Modaplex device comprising at least one excitation source and least one detector for detecting the fluorophore labeled nucleic acids, at least one processor, a least one microchip and at least one interface for digital data transmission, wherein the processor comprises at least one program controlling and operating the method according to any one of the claims claim 1 to 8 and/or the method of claims 17 to 19.
